# EUROPEAN PATENT SPECIFICATION

(11) **EP 1 385 541 B1**
(45) Date of publication and mention of the grant of the patent: **18.06.2008**
(21) Application number: 01925021.6
(22) Date of filing: 13.04.2001
(51) Int. Cl.: A61K 39/39, A61P 31/00

(54) **IMMUNOSTIMULANT COMPOSITIONS COMPRISING AN AMINOALKYL GLUCOSAMINIDE PHOSPHATE AND QS-21**
IMMUNOSTIMULIERENDE ZUSAMMNENSETZUNGEN DIE AMINOALKYL GLUCOSAMINIDEPHOSPHAT UND QS-21 ENTHALTEN
COMPOSITIONS IMMUNOSTIMULANTES COMPRENANT UN PHOSPHATE D'AMINOALKYL GLUCOSAMINIDE ET DU QS-21

(30) Priority: 13.04.2000 US 196846 P
(43) Date of publication of application: 04.02.2004
(73) Proprietor: CORIXA CORPORATION, Seattle, WA 98104 (US); Antigenics Inc., Woburn, MA 01801 (US)
(72) Inventor: MOSSMAN, Sally, Seattle, WA 98107 (US); EVANS, Lawrence, Seattle, WA 98126-3037 (US)
(74) Representative: Paget, Hugh Charles Edward
(86) International application number: PCT/US2001/012182
(87) International publication number: WO 2001/078777

(56) References cited:
- WO-A-95/17210
- WO-A-96/33739
- WO-A-98/50399
- WO-A-98/57659
- WO-A-99/40188
- JOHNSON D A ET AL: "SYNTHESIS AND BIOLOGICAL EVALUATION OF A NEW CLASS OF VACCINE ADJUVANTS: AMINOALKYL GLUCOSAMIDINE 4-PHOSPHATES (AGPS)" BIOORGANIC & MEDICINAL CHEMISTRY LETTERS, OXFORD, GB, vol. 9, no. 15, 1999, pages 2273-2278, XP002952215 ISSN: 0960-894X

## Description

### FIELD OF THE INVENTION

The present invention relates generally to vaccine formulations, to methods for their production and to their use in prophylactic and/or therapeutic vaccination. More particularly, the present invention relates to an adjuvant system comprising QS-21 in combination with an aminoalkyl glucosaminide phosphate comprising 2-[(R)-3-Tetradecanoyloxytetradecanoylamino]ethyl 2-Deoxy-4-O-phosphono-3-O-[(R)-3-tetradecanoyoxytetradecanoyl]-2-[(R)-3-tetradecanoyoxytetradecanoylamino]-β-D-glucopyranoside triethylammonium salt.

### BACKGROUND OF THE INVENTION

Humoral immunity and cell-mediated immunity are the two major branches of the mammalian immune response. Humoral immunity involves the generation of antibodies to foreign antigens. Antibodies are produced by B-lymphocytes. Cell-mediated immunity involves the activation of T-lymphocytes which either act upon infected cells bearing foreign antigens or stimulate other cells to act upon infected cells. Both branches of the mammalian immune system are important in fighting disease. Humoral immunity is the major line of defense against bacterial pathogens. In the case of viral disease, the induction of cytotoxic T lymphocytes (CTLs) appears to be crucial for protective immunity. Thus, an effective vaccine preferably stimulates both branches of the immune system to protect against disease.

Vaccines present foreign antigens from disease causing agents to a host so that the host can mount a protective immune response. Often, vaccine antigens are killed or attenuated forms of the microbes which cause the disease. The presence of non-essential components and antigens in these killed or attenuated vaccines has encouraged considerable efforts to refine vaccine components including developing well-defined synthetic antigens using chemical and recombinant techniques. The refinement and simplification of microbial vaccines, however, has led to a concomitant loss in potency. Low-molecular weight synthetic antigens, though devoid of potentially harmful contaminants, are often not sufficiently immunogenic by themselves. These observations have led investigators to add immune system stimulators known as adjuvants to vaccine compositions to potentiate the activity of the vaccine components.

Immune adjuvants are compounds which, when administered to an individual or tested in vitro, increase the immune response to an antigen in a subject to which the antigen is administered, or enhance certain activities of cells from the immune system. A number of compounds exhibiting varying degrees of adjuvant activity have been prepared and tested (see, for example, Shimizu *et al.* 1985, Bulusu *et al*. 1992, Ikeda *et al.* 1993, Shimizu *et al*. 1994, Shimizu *et al.* 1995, Miyajima *et al.* 1996). However, these and other prior adjuvant systems often display toxic properties, are unstable and/or have unacceptably low immunostimulatory effects.

Presently, the only adjuvant licensed for human use in the United States is alum, a group of aluminum salts (e.g., aluminum hydroxide, aluminum phosphate) in which vaccine antigens are formulated. Particulate carriers like alum reportedly promote the uptake, processing and presentation of soluble antigens by macrophages. Alum, however, is not without side-effects and is unfortunately limited to humoral (antibody) immunity only.

WO 96/33739 and WO 95/17210 describe the saponin QA-21 in combination with a sterol such as 3-deacylated monophosphoryl lipid A (MPL) and an antigen for use as a vaccine composition.

The discovery and development of effective adjuvant systems is essential for improving the efficacy and safety of existing and future vaccines. Thus there is a continual need for new and improved adjuvant systems, particularly those that drive both effector arms of the immune system, to better facilitate the development of a next generation of synthetic vaccines. The present invention fulfils these and other needs.

### SUMMARY OF THE INVENTION

In a first aspect of the present invention, there is provided an immunostimulant composition comprising QS-21 and RC-529 of formula I shown hereinafter, in an aqueous formulation.

In another aspect of the invention, there are provided vaccine compositions comprising the above immunostimulant compositions in combination with at least one antigen. The antigens may be derived from any of a variety of sources, and will most typically be derived from a bacterium or virus, or may be derived from antigens associated with cancer, autoimmune disorders, or a number of other mammalian diseases.

In another aspect of the present invention, there is provided the use of a composition of the invention in the manufacture of a medicament for treating a mammal suffering from or susceptible to a pathogenic infection, cancer or an autoimmune disorder

In another aspect of the present invention, the above medicament is for enhancing the immune response in a mammal, the medicament optionally including one or more antigens.

In another aspect of the invention, there are provided immunostimulant compositions comprising QS-21 and RC-529 in an aqueous formulation comprising one or more phospholipid surfactants.

### DETAILED DESCRIPTION OF THE INVENTION

Aminoalkyl glucosaminide phosphate (AGP) compounds generally comprise a 2-deoxy-2-amino-a-D-glucopyranose (glucosaminide) in glycosidic linkage with an aminoalkyl (aglycon) group. AGP compounds, and methods for their synthesis and use, are described generally in U.S. Patent 6,113,918 (which issued from U.S. Patent Application Serial No. 08/853,826), WO 98/50399, U.S. Patent Application Serial Nos. 09/074,720 and 09/439,839, and Johnson et al. (1999) Bioorg. Med. Chem. Lett. 9: 2273-2278.

The AGP compound of the subject invention, referred to as RC-529, is described structurally by Formula I below: 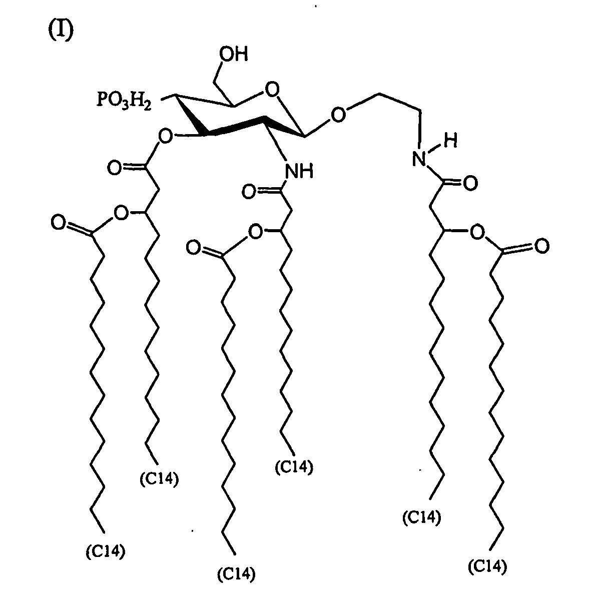 and pharmaceutically acceptable salts thereof.

One particularly preferred RC-529 compound is a 2-[(*R*)-3-tetradecanoyloxytetradecanoylamino]ethyl 2-deoxy-4-*O*-phosphono-3-*O*-[(*R*)-3-tetradecanoyoxytetradecanoyl]-2-[(*R*)-3-tetradecanoyoxytetradecanoylamino]-β-D-glucopyranoside triethylammonium salt.

The QS-21 saponin employed in the vaccine compositions of the present invention can be purified from Quillaja saponaria Molina bark, as described in U.S. Patent No. 5,057,540, the disclosure of which is incorporated herein by reference in its entirety. Briefly, aqueous extracts of Quillaja saponaria Molina bark are dialyzed against water and the dialyzed extract is lyophilized to dryness, extracted with methanol, and the methanol-soluble extract is further fractionated on silica gel chromatography and by reverse phase high pressure liquid chromatography (RP-HPLC). The individual saponins can then be separated by reverse phase HPLC. At least 22 peaks (denominated QA-1 to QA-22, also referred to herein as QS-1 to QS-21) are separable using this approach, with each peak corresponding to a carbohydrate peak and exhibiting a single band on reverse phase thin layer chromatography. The individual components can be specifically identified by their retention times on a C4 HPLC column, for example.

The substantially pure QS-21 saponin is characterized as having immune adjuvant activity, containing about 22% carbohydrate (as assayed by anthrone) per dry weight, having a UV absorption maxima of 205-210 nm, a retention time of approximately 51 minutes on RP-HPLC on a Vydac C₄ column having 5 µm particle size, 330 angstrom pore, 4.6 mm ID X 25 cm L in a solvent of 40 mM acetic acid in methanol/water (58/42; v/v) at a flow rate of 1 ml/min, eluting with 69 to 70% methanol from a Vydac C₄ column having 5 µm particle size, 330 angstrom pore, 10 mm ID X 25 cm L in a solvent of 40 mM acetic acid with gradient elution from 50 to 80% methanol, with a critical micellar concentration of about 0.03% (w/v) in water and 0.02% (w/v) in phosphate buffered saline, causing hemolysis of sheep red blood cells at concentrations of 25 µg/ml or greater, and containing the monosaccharides terminal rhamnose, terminal arabinose, terminal apiose, terminal xylose, 4-rhamnose, terminal glucose, terminal galactose, 2-fucose, 3-xylose, 3,4-rhamnose, and 2,3-glucuronic acid.

In one embodiment, the present invention provides pharmaceutical compositions, e.g., vaccine compositions, comprising a synergistic combination of RC-529 and QS-21. This adjuvant combination can be said to act in a synergistic fashion because it has an effect that is larger than the sum of the separate effects of each adjuvant. For example, this adjuvant system can synergistically enhance the immune responses to a co-administered antigen. The synergy between these two adjuvant-types for CTL induction has important implications for the use of recombinant molecules as vaccines for induction of CTL-mediated immunity.

Induction of CTL is typically seen when a target antigen is synthesized intracellularly (e.g. in infections by viruses, intracellular bacteria, or in tumors), because peptides generated by proteolytic breakdown of the antigen can enter the appropriate processing pathway, leading to presentation in association with class I molecules on the cell membrane. However, in general, pre-formed soluble antigen does not reach this processing and presentation pathway, and does not elicit class I restricted CTL. Therefore conventional non-living vaccines, while eliciting antibody and T helper responses, are not generally effective in inducing CTL-mediated immunity. The adjuvant combinations herein overcome this limitation of vaccines based on recombinant proteins, and induce a wider spectrum of immune responses.

The disclosed adjuvant systems comprising RC-529 and QS-21 can also enhance interferon (IFN) gamma production. IFN-gamma secretion is associated with protective responses against intracellular pathogens, including parasites, bacteria and viruses. Activation of macrophages by IFN-gamma enhances intracellular killing of microbes and increases expression of Fc receptors. Direct cytotoxicity may also occur, especially in synergism with lymphotoxin (another product of TH1 cells). IFN-gamma is also both an inducer and a product of NK cells, which are major innate effectors of protection. TH1 type responses, either through IFN-.gamma. or other mechanisms, provide preferential help for IgG2a immunoglobulin isotypes.

Thus, the adjuvant systems of the invention are particularly advantageous in making and using vaccine compositions to induce active immunity towards antigens in mammals, preferably in humans. Vaccine preparation is a well developed art and general guidance in the preparation and formulation of vaccines is readily available from any of a variety of sources. One such example is New Trends and Developments in Vaccines, edited by Voller et al., University Park Press, Baltimore, Md., U.S.A. 1978.

The optimal amount of a given vaccine composition to be administered will vary, however such information is easily determined using standard procedures. For example, the immunogenic activity of a given amount of a vaccine composition of the present invention can be determined by monitoring the increase in titer of antibody against the antigen used in the vaccine composition (Dalsgaard, K. Acta Veterinia Scandinavica 69:1-40 (1978)). Another common method involves injecting CD-1 mice intradermally with various amounts of a vaccine composition, later harvesting sera from the mice and testing for anti-immunogen antibody, e.g., by ELISA. These and other similar approaches will be apparent to the skilled artisan.

The adjuvant system of the present invention exhibits strong adjuvant effects when administered over a wide range of dosages and a wide range of ratios. The ratio of QS-21:RC-529 will typically be on the order of 1:10 to 10:1; more typically about 1:5 to 5:1 and often substantially 1:1. Typically for human administration, QS-21 RC-529 will be present in a vaccine composition in the range 1 µg-100 µg, preferably 10 µg-50 µg per dose.

The amount of antigenic protein in each vaccine dose is generally selected as an amount which induces an immunoprotective response without significant adverse side effects in typical vaccines. Such amount will vary depending upon which specific immunogen is employed and how it is presented. Generally, it is expected that each dose will comprise about 1-1000 µg of protein, most typically about 2-100 µg, preferably about 5-50 µg. Of course, the dosage administered may be dependent upon the age, weight, kind of concurrent treatment, if any, and nature of the antigen administered.

The antigen can be derived and/or isolated from essentially any desired source. By way of illustration, the antigens can be derived from viral sources, such as influenza virus, feline leukemia virus, feline immunodeficiency virus, HIV-1, HIV-2, rabies, measles, hepatitis B, or hoof and mouth disease viruses. Illustrative antigens can also be derived from bacterial sources, such as anthrax, diphtheria, Lyme disease, malaria, tuberculosis, Leishmaniasis, T. cruzi, Ehrlichia, Candida etc., or from protozoans such as Babeosis bovis or Plasmodium. The antigen(s) will typically be comprised of natural or synthetic amino acids, e.g., in the form of peptides, polypeptides, or proteins, can be comprised of polysaccharides, or can be mixtures thereof. Illustrative antigens can be isolated from natural sources, synthesized by means of solid phase synthesis, or can be obtained by way of recombinant DNA techniques.

In another embodiment, the adjuvant system of the present invention is used in prophylactic and/or therapeutic cancer vaccine compositions. Cancer cells often have distinctive antigens on their surfaces, such as truncated epidermal growth factor, folate binding protein, epithelial mucins, melanoferrin, carcinoembryonic antigen, prostate-specific membrane antigen, HER2-neu, which are candidates for use in therapeutic cancer vaccines. Because tumor antigens are normal or related to normal components of the body, the immune system often fails to mount an effective immune response against those antigens to destroy the tumor cells. To achieve such a response, the adjuvant systems described herein can be utilized. As a result, exogenous proteins can enter the pathway for processing endogenous antigens, leading to the production of cytolytic or cytotoxic T cells (CTL). This adjuvant effect facilitates the production of antigen specific CTLs which seek and destroy those tumor cells carrying on their surface the tumor antigen(s) used for immunization. Illustrative cancer types for which this approach can be used include prostate, colon, breast, ovarian, pancreatic, brain, head and neck, melanoma, leukemia, lymphoma, etc.

In another embodiment of the invention, the adjuvant system of the present invention can be administered alone, i.e., without a co-administered antigen, to potentiate the immune system for treatment of chronic infectious diseases, especially in immune compromised patients. Illustrative examples of infectious diseases for which this approach may be employed for therapeutic or prophylactic treatment can be found in U.S. Pat. No. 5,508,310. Potentiation of the immune system in this way can also be useful as a preventative measure to limit the risks of nosocomial and/or post-surgery infections.

In another embodiment, the antigen present in the vaccine compositions is not a foreign antigen, rather it is a self antigen, e.g., the vaccine composition is directed toward an autoimmune disease such as type 1 diabetes, conventional organ-specific autoimmune diseases, neurological diseases, rheumatic diseases, psoriasis, connective tissue diseases, autoimmune cytopenias, and other autoimmune diseases. Such conventional organ specific autoimmunity may include thyroiditis (Graves+Hashimoto's), gastritis, adrenalitis (Addison's), ovaritis, primary biliary cirrhosis, myasthenia gravis, gonadal failure, hypoparathyroidism, alopecia, malabsorption syndrome, pernicious anemia, hepatitis, anti-receptor antibody diseases and vitiligo. Such neurological diseases may include schizophrenia, Alzheimer's disease, depression, hypopituitarism, diabetes insipidus, sicca syndrome and multiple sclerosis. Such rheumatic diseases/connective tissue diseases may include rheumatoid arthritis, systemic lupus erythematous (SLE) or Lupus, scleroderma, polymyositis, inflammatory bowel disease, dermatomyositis, ulcerative colitis, Crohn's disease, vasculitis, psoriatic arthritis, exfoliative psoriatic dermatitis, pemphigus vulgaris, Sjorgren's syndrome. Other autoimmune related diseases may include autoimmune uvoretinitis, glomerulonephritis, post myocardial infarction cardiotomy syndrome, pulmonary hemosiderosis, amyloidosis, sarcoidosis, aphthous stomatitis, and other immune related diseases, as presented herein and known in the related arts.

In one embodiment, the adjuvant system described herein is used in the preparation of DNA-based vaccine compositions. Illustrative vaccines of this type contain DNA encoding one or more polypeptide antigens, such that the antigen is generated *in situ.* The DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin)* that expresses an immunogenic portion of the polypeptide on its cell surface or secretes such an epitope. In one preferred embodiment, the DNA is introduced using a viral expression system (e.g., vaccinia or other pox virus, retrovirus, or adenovirus), which typically involves the use of a non-pathogenic (defective), replication competent virus. Illustrative systems are disclosed, for example, in Fisher-Hoch et al., Proc. Natl. Acad. Sci. USA 86:317-321, 1989; Flexner et al., Ann. N.Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993. Techniques for incorporating DNA into such expression systems are well known to those of ordinary skill in the art. Alternatively, the DNA may be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads that are efficiently transported into the cells. It will be apparent that a vaccine may comprise both a polynucleotide and a polypeptide component if desired.

Moreover, it will be apparent that a vaccine may contain pharmaceutically acceptable salts of the desired polynucleotide, polypeptide and/or carbohydrate antigens. For example, such salts may be prepared from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

While any suitable carrier known to those of ordinary skill in the art may be employed in the vaccine compositions of this invention, the type of carrier will typically vary depending on the desired mode of administration. Compositions of the present invention may be formulated for any appropriate manner of administration, including for example, topical, oral, nasal, intravenous, intracranial, intraperitoneal, intradermal, subcutaneous or intramuscular administration. For parenteral administration, such as subcutaneous injection, the carrier will often comprise water, saline, alcohol, a fat, a wax or a buffer. For oral administration, the above carriers are often used, or a solid carrier such as mannitol, lactose, starch, magnesium stearate, sodium saccharine, talcum, cellulose, glucose, sucrose, and magnesium carbonate, can also be employed. Biodegradable microspheres (e.g., polylactate polyglycolate) may also be employed as carriers for the compositions of this invention. Suitable biodegradable microspheres are disclosed, for example, in U.S. Patent Nos. 4,897,268; 5,075,109; 5,928,647; 5,811,128; 5,820,883; 5,853,763; 5,814,344 and 5,942,252. Modified hepatitis B core protein carrier systems are also suitable, such as those described in WO/99 40934, and references cited therein. One may also employ a carrier comprising particulate-protein complexes, e.g., as described in U.S. Patent No. 5,928,647, the disclosure of which is incorporated herein by reference in its entirety, which are capable of inducing a class I-restricted cytotoxic T lymphocyte responses in a host.

In one illustrative embodiment, the vaccine formulations are administered to the mucosae, in particular to the oral cavity, and preferably to a sublingual site, for eliciting an immune response. Oral cavity administration may be preferred in many instances over traditional parenteral delivery due to the ease and convenience offered by noninvasive administration techniques. Moreover, this approach further provides a means for eliciting mucosal immunity, which can often be difficult to achieve with traditional parenteral delivery, and which can provide protection from airborne pathogens and/or allergens. An additional advantage of oral cavity administration is that patient compliance may be improved with sublingual vaccine delivery, especially for pediatric applications, or for applications traditionally requiring numerous injections over a prolonged period of time, such as with allergy desensitization therapies.

The vaccine compositions can also comprise buffers (e.g., neutral buffered saline, phosphate buffered saline or phosphate buffers w/o saline), carbohydrates (e.g., glucose, mannose, sucrose or dextrans), mannitol, proteins, polypeptides or amino acids such as glycine, antioxidants, bacteriostats, chelating agents such as EDTA or glutathione, adjuvants (*e*.*g*., aluminum hydroxide), solutes that render the formulation isotonic, hypotonic or weakly hypertonic with the blood of a recipient, suspending agents, thickening agents and/or preservatives. Alternatively, compositions of the present invention may be formulated as a lyophilizate. The compositions can also be encapsulated within liposomes using well known technology.

For certain applications, an aqueous formulation of RC-529 and QS-21 provides unexpectedly strong adjuvant activity. Therefore, in one embodiment, the vaccine composition is an aqueous formulation comprising one or more surfactants. For example, the composition can be in the form of a micellar dispersion comprising at least one suitable surfactant, e.g., a phospholipid surfactant. Illustrative examples of phospholipids include diacyl phosphatidyl glycerols, such as dimyristoyl phosphatidyl glycerol (DPMG), dipalmitoyl phosphatidyl glycerol (DPPG), and distearoyl phosphatidyl glycerol (DSPG), diacyl phosphatidyl cholines, such as dimyristoyl phosphatidylcholine (DPMC), dipalmitoyl phosphatidylcholine (DPPC), and distearoyl phosphatidylcholine (DSPC); diacyl phosphatidic acids, such as dimyristoyl phosphatidic acid (DPMA), dipalmitoyl phosphatidic acid (DPPA), and distearoyl phosphatidic acid (DSPA); and diacyl phosphatidyl ethanolamines such as dimyristoyl phosphatidyl ethanolamine (DPME), dipalmitoyl phosphatidyl ethanolamine (DPPE) and distearoyl phosphatidyl ethanolamine (DSPE).

Typically, a surfactant:adjuvant molar ratio in an aqueous formulation will be from about 10:1 to about 1:10, more typically from about 5:1 to about 1:5, however any effective amount of surfactant may be used in an aqueous formulation to best suit the specific objectives of interest.

In another embodiment, the composition is an emulsion, such as a water-in-oil emulsion or an oil-in water emulsion. Such emulsions are generally well known to those skilled in this art.

The adjuvant system of the present invention can be employed as the sole adjuvant system, or alternatively, can be administered together with other adjuvants or immunoeffectors. By way of illustration, such adjuvants can include oil-based adjuvants (for example, Freund's Complete and Incomplete), liposomes, mineral salts (for example, AlK(SO₄)₂, AlNa(SO₄)₂, AlNH₄(SO₄), silica, alum, Al(OH)₃, Ca₃(PO₄)₂, kaolin, and carbon), polynucleotides (for example, poly IC and poly AU acids), polymers (for example, non-ionic block polymers, polyphosphazenes, cyanoacrylates, polymerase-(DL-lactide-co-glycoside), among others, and certain natural substances (for example, lipid A and its derivatives, wax D from Mycobacterium tuberculosis, as well as substances found in Corynebacterium parvum, Bordetella pertussis, and members of the genus Brucella), bovine serum albumin, diphtheria toxoid, tetanus toxoid, edestin, keyhole-limpet hemocyanin, Pseudomonal Toxin A, choleragenoid, cholera toxin, pertussis toxin, viral proteins, and eukaryotic proteins such as interferons, interleukins, or tumor necrosis factor. Such proteins may be obtained from natural or recombinant sources according to methods well known to those skilled in the art. When obtained from recombinant sources, the adjuvant may comprise a protein fragment comprising at least the immunostimulatory portion of the molecule. Other known immunostimulatory macromolecules which can be used in the practice of the invention include, but are not limited to, polysaccharides, tRNA, non-metabolizable synthetic polymers such as polyvinylamine, polymethacrylic acid, polyvinylpyrrolidone, mixed polycondensates (with relatively high molecular weight) of 4',4-diaminodiphenylmethane-3,3'-dicarboxylic acid and 4-nitro-2-aminobenzoic acid (See Sela, M., Science 166:1365-1374 (1969)) or glycolipids, lipids or carbohydrates.

In one embodiment, the adjuvant system is preferably designed to induce an immune response predominantly of the Th1 type. High levels of Th1-type cytokines (e.g., IFN-γ, TNFα, IL-2 and IL-12) tend to favor the induction of cell mediated immune responses to an administered antigen. In contrast, high levels of Th2-type cytokines (e.g., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, *Ann. Rev. Immunol. 7*:145-173, 1989.

For example, additional adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Corixa Corporation (Seattle, WA; *see* US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Other illustrative adjuvants that can be included in the vaccine compositions include Montanide ISA 720 (Seppic, France), SAF (Chiron, California, United States), ISCOMS (CSL), MF-59 (Chiron), Detox (Corixa, Hamilton, MT).

The compositions described herein may be administered as part of a sustained release formulation (i.e., a formulation such as a capsule, sponge or gel (composed of polysaccharides, for example) that effects a slow release of compound following administration). Such formulations may generally be prepared using well known technology (*see, e.g.,* Coombes et al., Vaccine 14:1429-1438, 1996) and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. Such carriers include microparticles of poly(lactide-co-glycolide), polyacrylate, latex, starch, cellulose, dextran and the like. Other delayed-release carriers include supramolecular biovectors, which comprise a non-liquid hydrophilic core (*e*.*g*., a cross-linked polysaccharide or oligosaccharide) and, optionally, an external layer comprising an amphiphilic compound, such as a phospholipid (*see e.g.,* U.S. Patent No. 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented.

### EXAMPLES

The following examples are offered to illustrate, but not to limit the claimed invention.

This example demonstrates synergy between QS-21 adjuvant when combined with RC-529 adjuvant, such that greater levels of CTL activity and interferon-gamma secretion are induced with the combination than by either adjuvant alone, or in the absence of adjuvant. This experiment employed a recombinant polypeptide antigen from M. tuberculosis, referred to as rDPV, to immunize C57BL/6 mice subcutaneously. Briefly, groups of four female 6-8 week old C57BL/6 mice were immunized subcutaneously with 5ug rDPV combined with 10ug 529, 10ug QS-21 or a combination the two, formulated in both aqueous (AF) and oil emulsion (SE) formulations. The RC-529 aqueous formulations comprise DPPC surfactant, in which the DPPC:529 molar ratio is about 8:1. Additional mice received the equivalent dose of antigen formulated in adjuvant combinations comprising MPL (Corixa Corp., Seattle, WA) and QS-21 in aqueous and oil emulsion formulations. Control mice were immunized with PBS. Immunizations were performed at weeks 0,3 and 7, and spleens were harvested 2 weeks later. Single cell suspensions of splenocytes were stimulated in vitro with EL-4 cells stably transduced to express DPV. Thirteen days later these cells were assayed for CTL activity against EL-4-DPV by standard chromium release techniques. Additional fresh splenocytes were stimulated in vitro with 5ug/ml rDPV and supernatants were harvested 3 days later and assayed for IFN-g by ELISA. The results of the above experiments are summarized in Tables 1 and 2 below.

Table 1 illustrates interferon -gamma secretion from splenocytes of immunized mice following stimulation in vitro with 5µg/ml recombinant DPV protein. Concentration of IFN-γ was measured in 3-day supernatants by ELISA, and is expressed as mean concentration for groups of four mouse spleens.

**TABLE 1**

| **Immunogen** | **Interferon-gamma secretion (pg/ml)** |
|---|---|
| rDPV | 723.50 |
| rDPV + MPL-AF | 702.34 |
| rDPV + MPL-SE | 2861.04 |
| rDPV + 529-AF | 538.75 |
| rDPV + 529-SE | 14242.53 |
| rDPV + QS-21 | 831.73 |
| rDPV + QS-21 + MPL-AF | 25301.44 |
| rDPV + QS-21 + MPL-SE | 2896.08 |
| rDPV + QS-21 + 529-AF | 34294.48 |
| rDPV + QS-21 + 529-SE | 13275.99 |
| saline | 911.90 |

Table 2 shows CTL activity of splenocytes stimulated for 13 days in vitro with EL-4 cells stably expressing DPV. Percent specific lysis (chromium release) is expressed as the mean of four mouse spleens per group, with background lysis against EL-4 cells. subtracted, at an effector to target ratio of 100:1.

**TABLE 2**

| **Immunogen** | **% Specific Lysis** |
|---|---|
| rDPV | 8.1 |
| rDPV + MPL-AF | 10.5 |
| rDPV + MPL-SE | 13.6 |
| rDPV + 529-AF | 10.4 |
| rDPV + 529-SE | 14.0 |
| rDPV + QS-21 | 9.7 |
| rDPV + MPL-AF + QS-21 | 31.8 |
| rDPV + MPL-SE + QS-21 | 16.2 |
| rDPV + 529-AF + QS-21 | 32.7 |
| rDPV + 529-SE + QS-21 | 13.2 |
| Saline | 11.9 |

## Claims

1. An immunostimulant composition in an aqueous formulation comprising QS-21 and RC-529 of formula I: 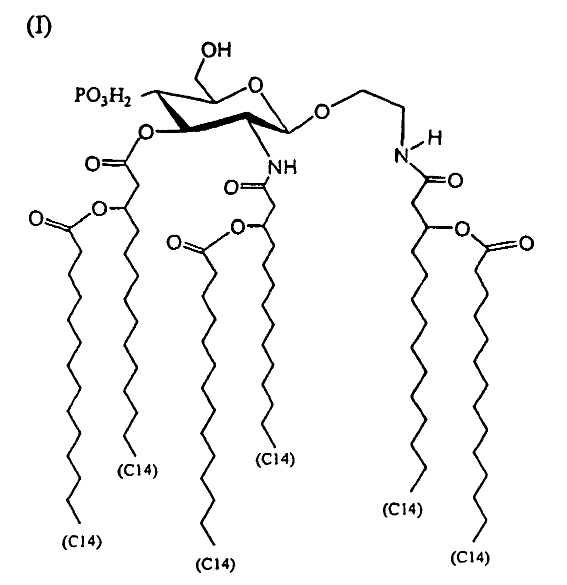 and pharmaceutically acceptable salts thereof.

2. The composition of claim 1 wherein the aqueous formulation comprises one or more surfactants.

3. The composition of claim 1 wherein the aqueous formulation comprises one or more phospholipid surfactants.

4. The composition of claim 3 wherein the surfactant is selected from the group consisting of diacyl phosphatidyl glycerols, diacyl phosphatidyl cholines, diacyl phosphatidic acids and diacyl phosphatidyl ethanolamines.

5. The composition of claim 3 wherein the surfactant is selected from the group consisting of dimyristoyl phosphatidyl glycerol (DPMG), dipalmitoyl phosphatidyl glycerol (DPPG), distearoyl phosphatidyl glycerol (DSPG), dimyristoyl phosphatidylcholine (DPMC), dipalmitoyl phosphatidylcholine (DPPC), distearoyl phosphatidylcholine (DSPC), dimyristoyl phosphatidic acid (DPMA), dipalmitoyl phosphatidic acid (DPPA), distearoyl phosphatidic acid (DSPA), dimyristoyl phosphatidyl ethanolamine (DPME), dipalmitoyl phosphatidyl ethanoline (DPPE) and distearoyl phosphatidyl ethanolamine (DSPE).

6. The composition of claim 1 further comprising at least one antigen.

7. The composition of claim 6 wherein the antigen is derived from the group consisting of Herpes Simplex Virus type 1, Herpes Simplex Virus type 2, Human cytomegalovirus, HIV, Hepatitis A, B, C or E, Respiratory Syncytial virus, human papilloma virus, Influenza virus, Tuberculosis, Leishmaniasis, T.Cruzi, Ehrlichia, Candida, Salmonella, Neisseria, Borrelia, Chlamydia, Bordetella, Plasmodium and Toxoplasma.

8. The composition of claim 7 wherein the antigen is a human tumour antigen.

9. The composition of claim 10 wherein the human tumour antigen is derived from a prostate, colon, breast, ovarian, pancreatic, brain, head and neck, melanoma, leukaemia or lymphoma cancer.

10. The composition of claim 7 wherein the antigen is a self-antigen.

11. The composition of claim 10 wherein the self antigen is an antigen associated with a an autoimmune disease.

12. The composition of claim 11 wherein the autoimmune disease is type 1 diabetes, multiple sclerosis, myasthenia gravis, rheumatoid arthritis or psoriasis.

13. The composition of claim 1 wherein the QS-21 and RC-529 are administered at a ratio of QS-21:RC-529 from about 1:10 to about 10:1.

14. The composition of claim 1 wherein the QS21 and RC-529 are administered at a ratio of QS-21:RC-529 from about 2.5:1 to about 1:2.5.

15. The composition of any one of claims 1 to 14 wherein the RC-529 of formula I is a 2-[(R)-3-tetradecanoyloxytetradecanoylamino]ethyl 2-deoxy-4-*O*-phosphono-*O*-[(R)-3-tetradecanoyloxytetradecanoyl]-2-[(R)-3-tetradecanoyloxytetradecanoylamino]-β-D-glucopyranoside triethylammonium salt.

16. A composition according to any one of claims 1 to 15 for use in therapy.

17. The use of a composition according to any one of claims 1 to 15 in the manufacture of a medicament for the treatment of a mammal suffering from or susceptible to a pathogenic infection, cancer or an autoimmune disorder.

18. The use of a composition according to any one of claims 1 to 15 in the manufacture of a medicament for enhancing the immune response in a mammal.

## Patentansprüche

1. Eine immunostimulierende Zusammensetzung in einer wässrigen Formulierung, die QS-21 und RC-529 nach Formel (I) 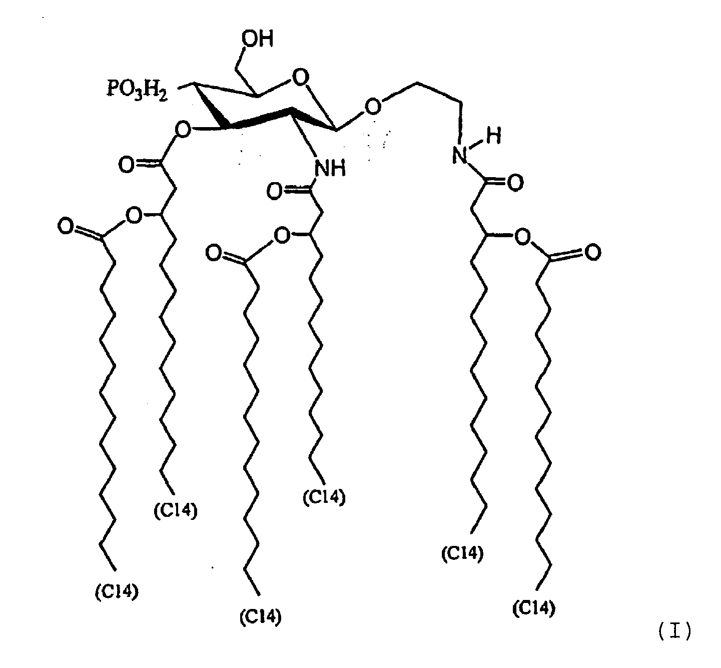 und pharmazeutisch geeignete Salze davon umfasst.

2. Die Zusammensetzung nach Anspruch 1, wobei die wässrige Formulierung ein oder mehrere Tenside umfasst.

3. Die Zusammensetzung nach Anspruch 1, wobei die wässrige Formulierung ein oder mehrere Phospholipid-Tenside umfasst.

4. Die Zusammensetzung nach Anspruch 3, wobei das Tensid aus der Gruppe, die aus Diacylphosphatidylglycerolen, Diacylphosphatidylcholinen, Diacylphosphatidsäuren und Diacylphosphatidylethanolaminen besteht, ausgewählt wird.

5. Die Zusammensetzung nach Anspruch 3, wobei das Tensid aus der Gruppe, die aus Dimyristoylphosphatidylglycerol (DPMG), Dipalmitoylphosphatidylglycerol (DPPG), Distearoylphosphatidylglycerol (DSPG), Dimyristoylphosphatidylcholin (DPMC), Dipalmitoylphosphatidylcholin (DPPC), Distearoylphosphatidylcholin (DSPC); Dimyristoylphosphatidsäure (DPMA), Dipalmitoylphosphatidsäure (DPPA), Distearoylphosphatidsäure (DSPA); Dimyristoylphosphatidylethanolamin (DPME), Dipalmitoylphosphatidylethanolamin (DPPE) und Distearoylphosphatidylethanolamin (DSPE) besteht, ausgewählt wird.

6. Die Zusammensetzung nach Anspruch 1, die weiter mindestens ein Antigen umfasst.

7. Die Zusammensetzung nach Anspruch 6, wobei das Antigen sich von der Gruppe, die aus Herpes Simplex Virus Typ 1, Herpes Simplex Virus Typ 2, humanem Cytomegalovirus, HIV, Hepatitis A, B, C oder E, Respiratory-Syncytial-Virus, humanem Papillomavirus, Grippevirus, Tuberkulose, Leishmaniose, T. Cruzi, Ehrlichia, Candida, Salmonella, Neisseria, Borrelia, Chlamydia, Bordetella, Plasmodium und Toxoplasma besteht, ableitet.

8. Die Zusammensetzung nach Anspruch 7, wobei das Antigen ein humanes Tumorantigen ist.

9. Die Zusammensetzung nach Anspruch 8, wobei sich das humane Tumorantigen von Prostata-, Kolon-, Brust-, Eierstock-, Pankreas-, Gehirn-, Kopf-Hals-, Melanom, Leukämie- oder Lymphomkrebs ableitet.

10. Die Zusammensetzung nach Anspruch 7, wobei das Antigen ein Autoantigen ist.

11. Die Zusammensetzung nach Anspruch 10, wobei das Autoantigen ein Antigen ist, das mit einer Autoimmunerkrankung verbunden ist.

12. Die Zusammensetzung nach Anspruch 11, wobei die Autoimmunerkrankung Typ 1 Diabetes, Multiple Sklerose, Myasthenia Gravis, rheumatoide Arthritis oder Psoriasis ist.

13. Die Zusammensetzung nach Anspruch 1, wobei das QS-21 und RC-529 in einem Verhältnis von QS-21:RC-529 von ungefähr 1:10 bis ungefähr 10:1 verabreicht werden.

14. Die Zusammensetzung nach Anspruch 1, wobei das QS-21 und RC-529 in einem Verhältnis von QS-21:RC-529 von ungefähr 2.5:1 bis ungefähr 1:2.5 verabreicht werden.

15. Die Zusammensetzung nach einem der Ansprüche 1 bis 14, wobei das RC-529 der Formel I ein 2-[(R)-3-Tetradecanoyloxytetradecanoylamino]ethyl-2-deoxy-4-O-phosphono-3-O-[(R)-3-tetradecanoyloxytetradecanoyl]-2-[(R)-3-tetradecanoyloxytetradecanoylamino]-β-D-glucopyranosidtriethylammonium-Salz ist.

16. Eine Zusammensetzung gemäß einem der Ansprüche 1 bis 15 für die Verwendung in der Therapie.

17. Die Verwendung einer Zusammensetzung gemäß einem der Ansprüche 1 bis 15 für die Herstellung eines Medikaments zur Behandlung eines Säugetiers, das an einer pathogenen Infektion, Krebs oder einer Autoimmunkrankheit leidet oder dafür anfällig ist.

18. Die Verwendung einer Zusammensetzung gemäß einem der Ansprüche 1 bis 15 für die Herstellung eines Medikaments zur Steigerung der Immunantwort in einem Säugetier.

## Revendications

1. Une composition immunostimulante dans une formulation aqueuse comportant le QS-21 et le RC-529 selon la formule I : 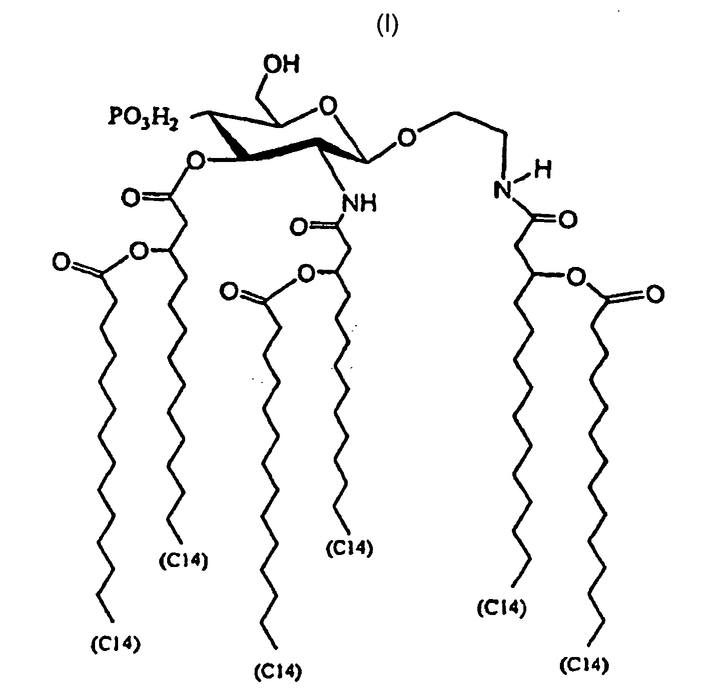 et des sels de celui-ci acceptables sur le plan pharmaceutique.

2. La composition selon la revendication 1 dans laquelle la formulation aqueuse comprend un ou plusieurs surfactants.

3. La composition selon la revendication 1 dans laquelle la formulation aqueuse comprend un ou plusieurs surfactants phospholipides.

4. La composition selon la revendication 3 dans laquelle le surfactant est choisi parmi le groupe constitué des glycérols de phosphatidyle de diacyle, des cholines de phosphatidyle de diacyle, des acides de phosphatidyle de diacyle, et des éthanolamines de phosphatidyle de diacyle.

5. La composition selon la revendication 3 dans laquelle le surfactant est choisi à partir du groupe constitué de glycérol de phosphatidyle de dimyristoyle (DPMG), de glycérol de phosphatidyle de dipalmitoyle (DPPG), de glycérol de phosphatidyle de distéaroyle (DSPG), de dimyristoyle phosphatidylcholine (DPMC), de dipalmitoyle phosphatidylcholine (DPPC), de distéaroyle phosphatidylcholine (DSPC), d'acide phosphatidique de dimyristoyle (DPMA), d'acide phosphatidique de dipalmitoyle (DPPA), d'acide phosphatidique de distéaroyle (DSPA), d'éthanolamine de phosphatidyle de dimyristoyle (DPME), d'éthanoline de phosphatidyle de dipalmitoyle (DPPE) et d'éthanolamine de phosphatidyle de distéaroyle (DSPE).

6. La composition selon la revendication 1 qui de plus comporte au moins un antigène.

7. La composition selon la revendication 6 dans laquelle l'antigène est dérivé à partir du groupe constitué du virus Herpes Simplex de type 1, du virus Herpes Simplex de type 2, du cytomégalovirus humain, de l'HIV, des hépatites A, B, C, ou E, du virus Respiratoire syncytial, du papilloma virus humain, du virus Influenza, de la tuberculose, de la leishmaniose, de T. Crusei, de Ehrlichia, de Candida, de Salmonella, de Neisseria, de Borrelia, de Chlamydia, de Bordetella, de Plasmodium et de Toxoplasme.

8. La composition selon la revendication 7 dans laquelle l'antigène est un antigène humain tumoral.

9. La composition selon la revendication 10 dans laquelle l'antigène humain est dérivé à partir d'un cancer de la prostate, du colon, du sein, de l'ovaire, du pancréas, du cerveau, de la tête et du cou, d'un mélanome, d'une leucémie ou d'un lymphome.

10. La composition selon la revendication 7 dans laquelle l'antigène est un auto-antigène.

11. La composition selon la revendication 10 dans laquelle l'auto-antigène est un antigène associé à une maladie auto-immune.

12. La composition selon la revendication 11 dans laquelle la maladie auto-immune est un diabète de type 1, une sclérose en plaques, une myasthénie, une polyarthrite rhumatoïde ou un psoriasis.

13. La composition selon la revendication 1 dans laquelle le QS-21 et le RC-529 sont administré selon un ratio de QS-21 : RC-529 de 1 :10 à environ 10 :1.

14. La composition selon la revendication 1 dans laquelle le QS-21 et le RC-529 sont administré selon un ratio de QS-21 : RC-529 de environ 2,5 : 1 à environ 1 : 2,5.

15. La composition selon l'une quelconque des revendications de 1 à 14 dans laquelle le RC-529 de formule I est un sel de 2-[(R)-3-tétradécanoyloxytetradécanoylamino]éthyle 2-déoxy-4-O-phosphono-O-[(R)-3-tétradécanoloxytétradécanoyle]-2-[(R)-3-tétradécanoyloxytétradécanoylamino]-β-D-glucopyranoside triéthylammonium.

16. Une composition en accord avec l'une quelconque des revendications de 1 à 15 pour une utilisation thérapeutique.

17. L'utilisation de la composition en accord avec l'une quelconque des revendications de 1 à 15 dans la fabrication d'un médicament pour le traitement d'un mammifère soufrant ou suspect d'une pathologie infectieuse, cancéreuse ou auto-immune.

18. L'utilisation de la composition en accord avec l'une quelconque des revendications de 1 à 15 dans la fabrication d'un médicament capable d'améliorer la réponse immune d'un mammifère.
